(19) Europäisches Patentamt
European Patent Office
Office européen des brevets

(11) Veröffentlichungsnummer: **0 320 805 B1**

(12) **EUROPÄISCHE PATENTSCHRIFT**

(45) Veröffentlichungstag der Patentschrift: **04.03.92**

(51) Int. Cl.[5]: **A61K 31/445**, C07D 211/46

(21) Anmeldenummer: **88120554.6**

(22) Anmeldetag: **08.12.88**

# (54) Acylierte Benzilsäurederivate zur Beeinflussung des Harnblasen-Tonus.

(30) Priorität: **16.12.87 DE 3742580**

(43) Veröffentlichungstag der Anmeldung:
**21.06.89 Patentblatt 89/25**

(45) Bekanntmachung des Hinweises auf die Patenterteilung:
**04.03.92 Patentblatt 92/10**

(84) Benannte Vertragsstaaten:
**CH DE ES FR GB IT LI NL**

(56) Entgegenhaltungen:
**EP-A- 0 102 554**
**GB-A- 2 031 727**
**US-A- 2 816 895**

**AM. J. OBSTET. GYNECOL., Band 153, 1985, Seiten 619-622; U. ULMSTEN et al.: "The effect of terodiline treatment in women with motor urge incontinence. Results from a double-blind study and long-term treatment"**

**S. BUDAVARI et al.: "The Merck Index", 1989, Auflage 11, no. 9089: "Terodiline", Merck & Co., Inc., Rahway, NJ, US**

(73) Patentinhaber: **SCHAPER & BRÜMMER GMBH & CO. KG**
**Bahnhofstrasse 45**
**W-3320 Salzgitter 61(DE)**

(72) Erfinder: **Pein, Eckart, Dr. med.**
**Sohnreystrasse 16**
**W-3410 Northeim(DE)**
Erfinder: **Ritter, Helmut, Prof.Dr.Dipl.-Chem.**
**Bergische Universität Wuppertal Gaussstrasse 20**
**W-5600 Wuppertal(DE)**
Erfinder: **Laven, Reinhard, Dipl.-Ing.**
**Schaper & Brümmer GmbH & Co.KG Bahnhofstrasse 35**
**W-3320 Salzgitter 61(DE)**

(74) Vertreter: **Lins, Edgar, Dipl.-Phys. et al**
**Patentanwälte Gramm + Lins Theodor-Heuss-Strasse 2**
**W-3300 Braunschweig(DE)**

**EP 0 320 805 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Gegenstand der vorliegenden Erfindung ist die Verwendung von acylierten Benzilsäurederivaten in Form von α,α-Diphenyl-α-acyloxy-essigsäure-4-(N-alkyl-piperidyl)-estern für therapeutische Zwecke. Sie betrifft auch neue Benzilsäurederivate.

Auf der Basis der α,α-Diphenyl-essigsäuren sind zahlreiche Substanzen beschrieben worden, die als neurotrope bzw. myotrope Spasmolytika Eingang in die Arzneimitteltherapie gefunden haben. Eine gezielte, selektive Wirkung dieser Substanzen auf den Harnblasen-Tonus konnte bislang jedoch nicht zufriedenstellend aufgezeigt werden. Eine therapeutische Anwendung in dieser Richtung wurde bisher nur durch das veretherte Derivat Propiverin (α,α-Diphenyl-α, n-propoxyessigsäure-4-(1-methylpiperidyl)-ester) realisiert (Zbl. Pharm. 120 (1981), 12, Seiten 1219-1224). Diese Verbindung zeigt jedoch bei einigen Patienten eine unzureichende Wirksamkeit und kann wegen bestimmter Nebenwirkungen nicht allgemein verwendet werden. Darüber resultiert aus dem hydrophoben Charakter der Verbindung eine verringerte Selektivität im Zielbereich der Harnblase.

Weiterhin bewirkt die hohe myotrop-spasmolytische Eigenwirkung bisher eingesetzter cholinolytischer Verbindungen im Bereich der Harnblase nicht selten eine unzureichende Kontraktion des Blasenmuskels bei aktiver Miktion mit der Gefahr der Bildung von Restharn.

Als ein weiterer Nachteil einer ausgeprägten myotrop-spasmolytischen Aktivität ist die Absenkung des Blutdrucks zu sehen, wie sie für die Therapie mit Propiverin beschrieben wurde (M. Blau, U. Retzke, Zbl. Gynäkol. 100 (1984), 14, Seiten 981-987).

Der vorliegenden Erfindung liegt die Aufgabe zugrunde, eine Substanzgruppe anzugeben, die die geschilderten Nachteile nicht aufweist und für eine selektive Therapie hypertoner Funktionszustände im Bereich der Harnblase geeignet ist.

Diese Aufgabe wird erfindungsgemäß durch acylierte Benzilsäurederivate gemäß der allgemeinen Strukturformel

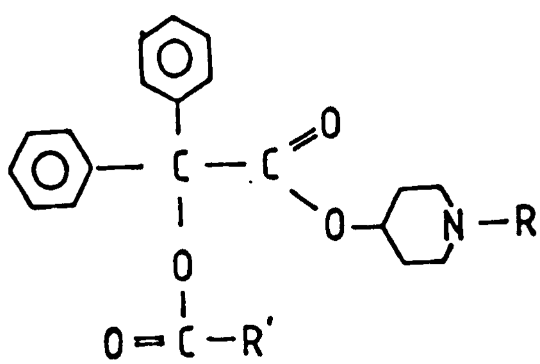

gelöst, wobei R für H, $CH_3$, $C_2H_5$, vorzugsweise $CH_3$ steht und R′ durch H, $NH_2$, Phenyl, das durch eine COOH-Gruppe substituiert sein kann, eine $(C_1\text{-}C_6)$Alkylgruppe, Hydroxy$(C_1\text{-}C_6)$alkylgruppe, Methoxy- oder Ethoxy-Alkylgruppe mit 0 oder 1 C-Atomen im Alkylteil, Amino$(C_1\text{-}C_6)$ alkylgruppe, die im Alkylrest durch eine Gruppe $-NH_2$, -COOH und/oder $-SCH_3$ substituiert sein kann, N-Acetylamino$(C_1\text{-}C_6)$alkylgruppe und Gruppen der Formel
-CH = CH-COOR″ oder

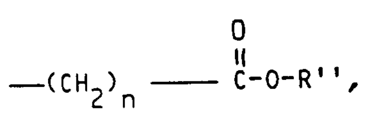

wobei n Werte von 0 bis 8 bevorzugt 0 bis 5 und besonders bevorzugt den Wert 2 annehmen kann und R″ H, $CH_3$, $C_2H_5$, $C_3H_7$, ein pharmakologisch unbedenkliches Kation, z.B. ein Alkali- (wie Li-, Na-, K-) Ca-, Aluminium-oder Ammonium-Ion sein kann, bedeutet. Wenn n den Wert 0 darstellt, so ist R′ eine Gruppe -COOR″, worin R″ wie vorstehend definiert ist.

Besonders bevorzugt sind solche Verbindungen, worin R′ die Gruppe $-(CH_2)_n-COOR''$ bedeutet, worin n = 1 und insbesondere 2 oder 3, und R″ wie vorstehend definiert ist. Im letzteren Falle stellt R″

vorzugsweise ein Wasserstoffatom oder ein pharmakologisch unbedenkliches Kation dar. Bevorzugt sind auch solche Verbindungen, worin R′ die Gruppe $CH_3$ oder $C_2H_5$ darstellt.

Bevorzugt sind auch solche Verbindungen, worin der Rest R die Methylgruppe darstellt.

Besonders geeignet im Sinne der vorliegenden Erfindung ist das Substrat α,α-Diphenyl-α (3-carboxypropionyloxy- oder 4-carboxybutyryloxy-)) essigsäure-4-(N-methyl- piperidyl)- ester (Succinylester bzw. Glutareste), sowie deren Salze mit pharmakologisch unbedenklichen Säuren. Es handelt sich um die Verbindung der allgemeinen Formel I, worin R die Methylgruppe darstellt und R′ $-(CH_2)_{2\ \mathrm{oder}\ 3}$ -COOR″ - (R″ = H) bedeutet. Diese Verbindungen liegen in der Betainstruktur vor. Sie wirken jedoch auch in Form der Ester (z. B. R″ = $CH_3$, $C_2H_5$ oder $C_3H_7$) oder der Salze (R″ = ein pharmakologisch verträgliches Kation). Sie können auch als Säureadditionssalze, wie nachstehend definiert, eingesetzt werden (wobei dann R″ bevorzugt H, $CH_3$, $C_2H_5$ oder $C_3H_7$ ist).

Die erfindungsgemäß verwendeten Verbindungen können als freie Basen, in der Betainform, wenn der Rest R″ in der Gruppe $-(CH_2)_n$-COOR″ ein H-Atom ist, oder in der Form ihrer Säureadditionssalze mit pharmakologisch unbedenklichen Säuren vorliegen. Derartige Säuren sind z.B. anorganische Säuren, wie Chlorwasserstoffsäure und Schwefelsäure oder organische Säuren, wie Polymersäuren, wie Carboxymethylcellulose.

Die erfindungsgemäß verwendeten Verbindungen, und insbesondere die vorstehend erwähnten bevorzugten Derivate sind besonders zur medikamentösen Therapie hypertoner Funktionszustände im Bereich der Harnblase durch selektive Hemmung parasympathisch gesteuerter Miktionsfrequenzen geeignet. Dadurch können Krankheitszustände wie hyperreflektorische Reizblase nach Bestrahlung sowie bei chronischer Zystitis behandelt werden. Als weitere Indikationen gelten die Drang-Inkontinenz, die Pollakisurie und die Nykturie. Die geringe myotrop-spasmolytische Aktivität des vorzugsweise verwendeten Succinylesters erscheint unter therapeutischen Gesichtspunkten als besonders vorteilhaft.

Die erfindungsgemäß verwendeten acylierten Benzilsäurederivate der allgemeinen Formel I, worin R′ den Rest $-(CH_2)_n$-COOR″ darstellt, sind neu. Die Erfindung betrifft daher auch neue α,α-Diphenyl-α-acyloxy-essigsäure-4-(N-alkyl-piperidyl)-ester der allgemeinen Formel Ia

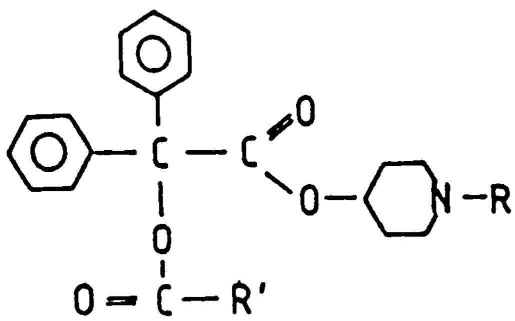

(Ia)

in der R für H, $CH_3$, $C_2H_5$, $C_3H_7$ und R′ für

$-(CH_2)_n$ -COOR″

steht,

wobei n eine ganze Zahl von 0 bis 8, bevorzugt 0 bis 5 oder 1 bis 3 und besonders bevorzugt 2 oder 3 ist und R″ H, $CH_3$, $C_2H_5$, $C_3H_7$, oder ein pharmakologisch verträgliches Kation, wie ein Alkali- (z. B. Li-, Na-, K-), Ca-, Al- oder Ammonium-Ion bedeutet, und deren Salze mit pharmakologisch verträglichen Säuren.

Der Rest R in der vorstehenden Formel Ia ist bevorzugt eine Methylgruppe. Bevorzugt sind die Derivate, worin n = 2 oder 3. Weiter bevorzugt sind die vorstehend zur Verwendung als bevorzugt definierten Derivate, die von der allgemeinen Formel Ia umfaßt werden, sowie deren Salze. Besonders bevorzugt sind die vorstehend erwähnten Succinyl- und Glutarester und deren Salze mit pharmakologisch verträglichen Säuren.

Die Herstellung der erfindungsgemäßen Verbindungen sowie der erfindungsgemäß verwendeten Verbindungen und ihrer Salze erfolgt durch O-Acylierung von α,α-Diphenyl-α-hydroxyessigsäure-4-(N-alkylpiperidyl)-ester mit geeigneten organischen Säuren bzw. von deren aktivierten Derivaten. Geeignete aktivierte Derivate sind z. B. das Disäurechlorid oder cyclische Anhydrid oder Alkyldiester der Kohlensäure. Eine erhaltene freie funktionelle Gruppe oder COOH-Gruppe wird gegebenenfalls in üblicher Weise in die

gewünschte Form umgewandelt, z. B. mit einem entsprechenden Alkohol verestert oder in das gewünschte Salz überführt. Der α-Hydroxyester kann nach J. Klosa und G. Delmar, J. für prakt. Chemie, 4., Bd 16, S 71ff (1962) hergestellt werden.

Geeignete Säuren für die O-Acylierung sind z.B.:

a) monofunktionelle Carbonsäuren, wie Ameisensäure, Essigsäure, Propionsäure, Buttersäure, Benzoesäure;

b) Hydroxysäuren, wie Hydroxyessigsäure, Milchsäure, Hydroxybuttersäure, Methoxyessigsäure, Ethoxyessigsäure;

c) Aminosäuren, wie Glycin, Alanin, Asparaginsäure, Methionin, Lysin oder

d) difunktionelle Säuren bzw. Dicarbonsäuren, wobei die nicht an der O-Acylierung beteiligte Carboxylgruppe als freie Säure, Methyl-, Ethyl, oder Propylester oder in Form eines Salzes, z. B. eines Alkali-(Li-, Na-, K-,), Ca-, Al- oder Ammoniumsalzes vorliegen kann. Beispiele für die Dicarbonsäure sind Kohlensäure, Carbamidsäure, Oxalsäure, Malonsäure, Bernsteinsäure, Glutarsäure, Adipinsäure, Sebazinsäure, Maleinsäure oder Phthalsäure. Die Säuren können z. B. in Form ihrer für die Acylierung geeigneten aktiven Derivate, wie Halogenide und die Dicarbonsäuren in Form ihrer Säureanhydride eingesetzt werden.

Die acylierten Benzilsäurederivate können auf verschiedene Weise verabreicht werden, z. B. auf oralem, peroralem, intravenösem und subcutanem Wege. Sie können in üblicher Weise formuliert werden, z.B. in wirksamen Dosen in Form eines Pulvers, als Tablette oder in wässriger bzw. in öliger Dispersion. Die Wirkstoffe können in Kombination mit weiteren therapeutischen aktiven Substanzen, wie Antibiotika, Beruhigungsmitteln oder Schmerzmitteln verwendet werden. Im übrigen dienen zur Formulierung übliche Hilfs- und Zusatzstoffe.

Die folgenden Beispiele sollen die Erfindung näher erläutern.

Herstellungsbeispiel 1:

3,25 g Benzilsäure-4(N-methylpiperidyl)-ester und 1,1 g Bersteinsäureanhydrid wurden in 50 ml Dioxan 8 Stunden unter Rückfluß erwärmt. Nach dem Abdestillieren des Lösungsmittels wurde aus Ethanol umkristallisiert. Man erhielt Kristalle von Fp 168 °C in einer Ausbeute von 80 %.

| Elementaranalyse | | |
|---|---|---|
| C | H | N |
| 67,75<br>67,72 | 6,40<br>6,67 | 3,29 (ber.)<br>3,42 (gef.) |

Formulierungsbeispiele:

| 1. Tabletten zu 200 mg | |
|---|---|
| Succinylester (α,α-Diphenoyl-α-(3-carboxy-propionyloxy)-4-(N-methylpiperidyl)-ester | 20 mg |
| mikrokristalline Zellulose (Avicel pH 102 der Fa. Atlas Chemicals) | 159 mg |
| quervernetzendes Polyvinylpyrrolidon (Polyplasdone XL der Fa. Antara Chemicals) | 20 mg |
| Magnesiumstearat | 1 mg |

| 2. Kapseln zu 300 mg | |
|---|---|
| Succinylester | 20 mg |
| Gelbes Bienenwachs | 10 mg |
| Sojabohnenöl | 10 mg |
| Planzenöl | 160 mg |
| Kapselhülle | 100 mg |

| 3. Injektionsampullen | |
|---|---|
| Succinylester<br>Phys. NaCl | 20 mg<br>ad 2 ml |

| 4. Infusionslösungen | |
|---|---|
| Succinylester<br>Phys. NaCl | 2 mg<br>ad 500 ml |

5. Flüssige orale Arzneiform

| | | |
|---|---|---|
| Succinylester | | 500 mg |
| Sorbit | | 10 g |
| Saccharin Natrium | | 0,050 g |
| Thanol 20 % (V/V) | ad | 100 ml |
| Aromaessenz in ausreichender Menge | | |

In den folgenden Beispielen wird die pharmakologische Wirksamkeit der bevorzugten Succinylester-Verbindung dargestellt, wodurch die Verwendung der erfindungsgemäß eingesetzten Verbindungen zur Behandlung hypertoner Funktionsstörungen der glatten Muskulatur des Urogenitalsystems veranschaulicht wird.

Beispiel 1

Die neurotrop-spasmolytische Wirkung der Substanzen wurde am isolierten Harnblasenmuskelpräparat des Meerschweinchens in vitro in üblicher Technik untersucht. Als Spasmodikum diente Carbachol. Carbachol wurde in einer Dosierung von 10 µg/ml dem Organbad zugesetzt, um eine maximale Kontraktion des Muskelpräparates zu erreichen.

Die zu testenden Verbindungen werden fünf Minuten nach Aktivierung des Muskelpräparates durch Carbachol in steigender Dosierung dem Organbad zugesetzt und aus den resultierenden Dosiswirkungskurven die entsprechenden $ED_{50}$-Werte ermittelt.

In der aufgezeichneten Versuchsanordnung zeigte sich, daß der Succinylester eine ausgeprägte, nahezu spezifisch neurotrop-spasmolytische Aktivität aufweist, die deutlich, um den Faktor 50, über der des Propiverins lag (s. Abbildung 1).

Beispiel 2

Gleichfalls am Modell des isolierten Harnblasenmuskelpräparates des Meerschweinchens wurde die myotrop-spasmolytische Aktivität beider Verbindungen vergleichend untersucht. Als Spasmodikum diente Bariumchlorid. Bariumchlorid wurde in einer Dosierung von 0,2 mg/ml dem Organbad zur Auslösung einer maximalen Kontraktion des Blasenmuskels zugesetzt. Während die myotrop-spasmolytische Aktivität des Propiverins ($ED_{50}$ 53,05 µg/ml) die Wirkungsstärke des Papaverins erreichte, zeigte der Succinylester eine um den Faktor 14 schwächere myotrop-spasmolytische Aktivität (s. Abbildung 2).

Beispiel 3

Zur weiteren Absicherung des Befundes erfolgte zusätzlich die Bestimmung der myotrop-spasmolytischen Aktivität von Propiverin und dem Succinylester am Modell der isolierten Taenia coli des Meerschweinchens in vitro.

Als Spasmodikum diente Bariumchlorid in einer Badkonzentration von 0,2 mg/ml.

Der $ED_{50}$-Wert für Propiverin lag bei 11,53 µg/ml und der $ED_{50}$-Wert für den Succinylester bei 80,62

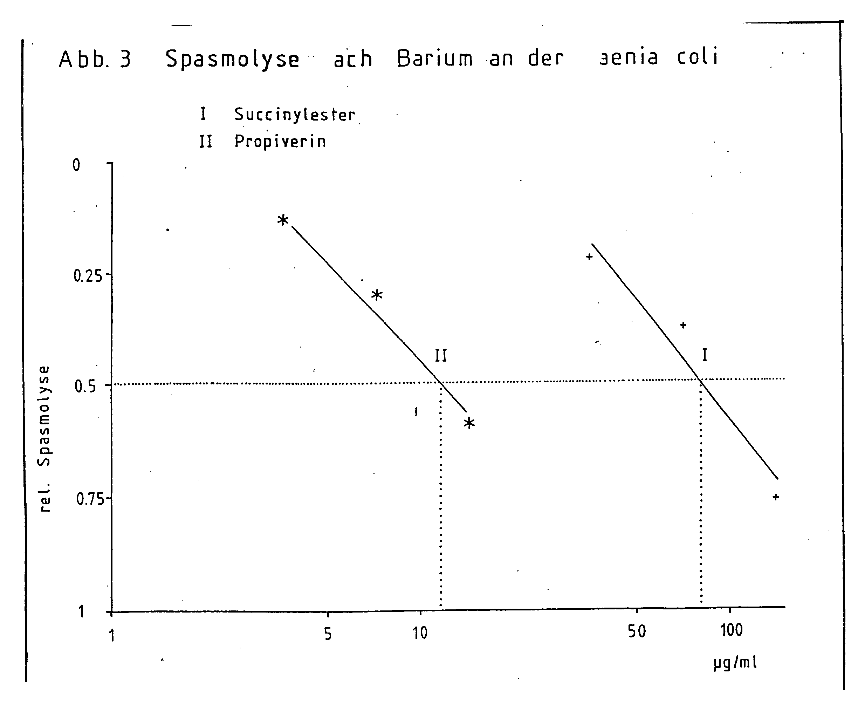

µg/ml, d.h., daß der Succinylester auch an diesem Versuchsmodell eine bedeutend geringere myotrop-spasmolytische Wirksamkeit als das Propiverin aufwies (s. Abbildung 3).

Herstellungsbeispiel 2:

Es wurde gearbeitet, wie im Herstellungsbeispiel 1 angegeben, jedoch unter Ersatz des Bernsteinsäureanhydrids durch Glutarsäureanhydrid. Der erhaltene Glutarester, der Formel Ia, worin R = $CH_3$ und R′ = $-(CH_2)_3-COOH$ zeigte im IR-Spektrum (KBr) 2 Ester-Peaks bei 1725 und 1735 $cm^{-1}$ und einen Carboxylat-Peak bei 1560 $cm^{-1}$.

Herstellungsbeispiel 3:

Die Acetylverbindung der Formel I, worin R = $CH_3$ und R′ = $CH_3$ wurde wie in J. Klosa und G. Delamar, J. für praktische Chemie, 4., Bd 16, S. 71 ff (1962) beschrieben, hergestellt.

Beispiel 4

Wie in den Beispielen 1 und 2 beschrieben, wurden die neurotrop-spasmolytische Wirkung und die myotrop-spasmolytische Wirkung der in den Herstellungsbeispielen 1, 2 und 3 beschriebenen Verbindungen, sowie der Vergleichsubstanz Propiverin bestimmt. Die Bedingungen und Ergebnisse sind in der nachfolgenden Tabelle angegeben. Es ist ersichtlich, daß bei den erfindungsgemäßen Verbindungen die neurotrop-spasmolytische Wirkung überwiegt, wobei bei der Vergleichsverbindung die unerwünschte myotrop-spasmolytische Wirkung überwiegt.

Tabelle

| | neurotrop- spasmolytische Wirkung: Ileum Carbachol 50 ng/ml | myotrop- spasmolytische Wirkung: Taenia coli Barium 200 µg/ml | |
|---|---|---|---|
| Vergleich | 59 ng | 12 µg | (EC 50) |
| Essigsäureester (Herstellungsbeispiel 3) | 5 ng | 76 µg | (EC 50) |
| Bernsteinsäureester (Herstellungsbeispiel 1) | 0.8 ng | 94 µg | (EC 50) |
| Glutarsäureester (Herstellungsbeispiel 2) | 1.5 ng | 229 µg | (EC 50) |

**Patentansprüche**

1. Arzneimittel, enthaltend acylierte Benzilsäurederivate in Form von α,α-Diphenyl-α-acyloxy-essigsäure-4-(N-alkyl-piperidyl)-estern der allgemeinen Formel

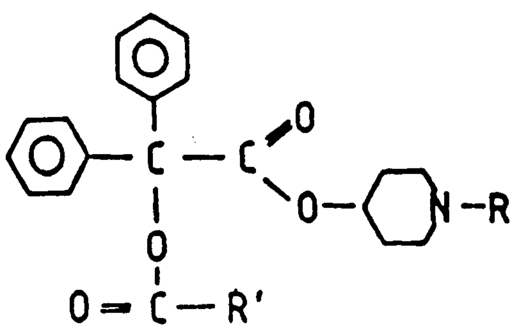

(I)

in der R für H, $CH_3$, $C_2H_5$, $C_3H_7$,

$R'$ für H, $NH_2$, Phenyl, das durch eine Carboxylgruppe substituiert sein kann, eine ($C_1$-$C_6$) Alkylgruppe, Hydroxy($C_1$-$C_6$)alkyl- gruppe, Methoxy-, Ethoxy-, Methoxymethyl- oder Ethoxymethylgruppe, Amino($C_1$-$C_6$) alkylgruppe, die im Alkylrest durch eine Gruppe -$NH_2$, -COOH und/oder -$SCH_3$ substituiert sein kann, N-Acetylamino($C_1$-$C_6$)alkylgruppe und eine Gruppe Gemäß der Formel

$-CH=CH-COOR''$ oder $-(CH_2)_n-COOR''$

steht,
wobei n eine ganze Zahl von 0 bis 8 ist und $R''$ H, $CH_3$, $C_2H_5$, $C_3H_7$ oder ein pharmakologisch verträgliches Kation bedeutet oder deren Salze mit pharmakologisch verträglichen Säuren.

**2.** Arzneimittel nach Anspruch 1, mit acylierten Benzilsäurederivaten der in Anspruch 1 definierten Formel I, worin R $CH_3$ ist, oder einem Salz davon mit einer pharmakologisch verträglichen Säure.

**3.** Arzneimittel nach Anspruch 1 oder 2, mit acylierten Benzilsäurederivaten der im Anspruch 1 definierten Formel I, worin $R'$ die Bedeutung von $-(CH_2)_n-COOR''$ hat, n 1, 2 oder 3 ist, und $R''$ wie in Anspruch 1 definiert ist, oder einem Salz davon mit einer pharmakologisch verträglichen Säure.

**4.** Arzneimittel nach Anspruch 1 enthaltend α,α-Diphenyl-α-(3-carboxy-propionyloxy)essigsäure-4-(N-methylpiperidyl)-ester, oder α, α-Diphenyl-α-(4-carboxy-butyryloxy)essigsäure-4-(N-methylpiperidyl)-ester und/oder α,α-Diphenyl-α-acetoxy-4-(N-methylpiperidyl)ester oder Salze davon mit pharmakologisch verträglichen Säuren.

**5.** Arzneimittel nach einem der Ansprüche 1 bis 4 zur therapeutischen Beeinflussung des Harnblasen-Tonus.

**6.** Benzilsäurederivate in Form von α,α-Diphenyl-α-acyloxy-essigsäure-4-(N-alkyl-piperidyl)-estern mit der Formel

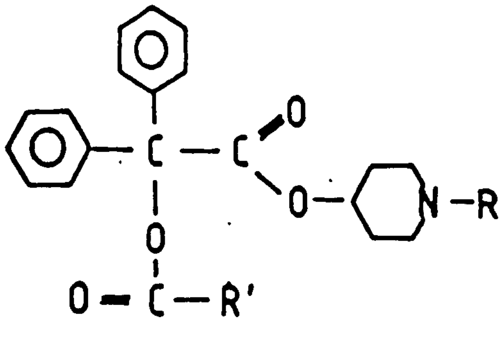

(I)

in der R für H, $CH_3$, $C_2H_5$, $C_3H_7$ und $R'$ für

$-(CH_2)_n-COOR''$

steht,
wobei n eine ganze Zahl von 0 bis 8 ist und $R''$ H, $CH_3$, $C_2H_5$, $C_3H_7$, oder ein pharmakologisch verträgliches Kation bedeutet und deren Salze mit pharmakologisch verträglichen Säuren.

**7.** Benzilsäurederivate nach Anspruch 6, worin n den Wert 0 bis 5, besonders 1 bis 3 und insbesondere den Wert 2 hat, und deren Salze mit pharmakologisch verträglichen Säuren.

**8.** Benzilsäurederivate nach Anspruch 6 oder 7, worin R $CH_3$ ist, und deren Salze mit pharmakologisch verträglichen Säuren.

**9.** α,α-Diphenyl-α-(3-carboxy-propionyloxy)essigsäure-4-(N-methylpiperidyl) ester und α,α-Diphenyl-α-(4-carboxy-butyryloxy)-essigsäure-4-(N-methylpiperidyl)ester und deren Salze mit pharmakologisch verträglichen säuren.

10. Verwendung der in den Ansprüchen 1 bis 5 und 6 bis 9 definierten Benzilsäurederivate zur therapeutischen Beeinflussung des Harnblasen-Tonus.

11. Verwendung der in den Ansprüchen 1 bis 5 und 6 bis 9 definierten Benzilsäurederivate zur Herstellung von Arzneimitteln für die therapeutische Beeinflussung des Harnblasen-Tonus.

**Claims**

1. Medicaments containing acylated benzilic acid derivatives in the form of the 4-(N-alkyl-piperidyl) esters of α,α-diphenyl-α-acyloxy-acet

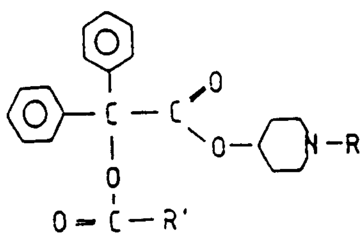

(I)

ic acid of the general formula in which R represents H, $CH_3$, $C_2H_5$, $C_3H_7$,

R' represents H; $NH_2$; phenyl which may be subsituted by a carboxyl group; a $(C_1$-$C_6)$-alkyl group, hydroxy-$(C_1$-$C_6)$-alkyl group, methoxy, ethoxy, methoxymethyl or ethoxymethyl group; amino-$(C_1$-$C_6)$-alkyl group which may be subsituted in the alkyl radical by a $-NH_2$, -COOH and/or $-SCH_3$ group; N-acetylamino-$(C_1$-$C_6)$-alkyl group or a group of the formula

-CH=CH-COOR" or $-(CH_2)_n$-COOR"

where n is an integer from 0 to 8 and R" signifies H, $CH_3$, $C_2H_5$, $C_3H_7$ or a pharmacologically acceptable cation, or salts thereof with pharmacologically acceptable acids.

2. Medicaments according to claim 1, having acylated benzilic acid derivatives of the formula I defined in claim 1 in which R is $CH_3$, or having a salt thereof with a pharmacologically acceptable acid.

3. Medicaments according to claim 1 or 2, having acylated benzilic acid derivatives of the formula I defined in claim 1 in which R' has the meaning $-(CH_2)_n$-COOR", n is 1, 2 or 3, and R" is as defined in claim 1, or having a salt thereof with a pharmacologically acceptable acid.

4. Medicaments according to claim 1, containing a 4-(N-methylpiperidyl) ester of α,α-diphenyl-α-(3-carboxylpropionyloxy)acetic acid, or a 4-(N-methylpiperidyl) ester of α,α-diphenyl-α-(4-carboxylbutyryloxy)acetic acid and/or a α,α-diphenyl-α-acetoxy-4-(N-methylpiperidyl) ester or salts thereof with pharmacologically acceptable acids.

5. Medicaments according to one of claims 1 to 4, for therapeutically affecting the tonus of the urinary bladder.

6. Benzilic acid derivatives in the form of 4-(N-alkylpiperidyl) esters of α,α-diphenyl-α-acyloxy-acetic acid of the formula

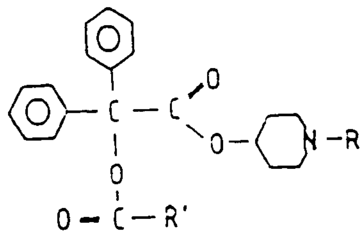

(I)

in which R represents H, $CH_3$, $C_2H_5$, $C_3H_7$ and R' represents

$-(CH_2)_n-COOR''$,

where n is an integer from 0 to 8 and R'' signifies H, $CH_3$, $C_2H_5$, $C_3H_7$ or a pharmacologically acceptable cation and salts thereof with pharmacologically acceptable acids.

**7.** Benzilic acid derivatives according to claim 6, in which n has the value 0 to 5, in particular 1 to 3 and most particularly 2, and salts thereof with pharmacologically acceptable acids.

**8.** Benzilic acid derivatives according to claim 6 or 7, in which R is $CH_3$, and salts thereof with pharmacologically acceptable acids.

**9.** 4-(N-methylpiperidyl) esters of $\alpha,\alpha$-diphenyl-$\alpha$-(3-carboxylpropionyloxy)acetic acid and 4-(N-methylpiperidyl) esters of $\alpha,\alpha$-diphenyl-$\alpha$-(4-carboxyl-butyryloxy)-acetic acid and salts thereof with pharmacologically acceptable acids.

**10.** Use of the benzilic acid derivatives defined in claims 1 to 5 and 6 to 9 for therapeutically affecting the tonus of the urinary bladder.

**11.** Use of the benzilic acid derivatives defined in claims 1 to 5 and 6 to 9 for manufacturing medicaments for therapeutically affecting the tonus of the urinary bladder.

## Revendications

**1.** Médicament contenant des dérivés d'acide benzylique acylés sous forme de 4-(N-alcoyl-pipéridyl)-esters d'acide $\alpha,\alpha$-diphényl-$\alpha$-acyloxy-acétique de formule générale

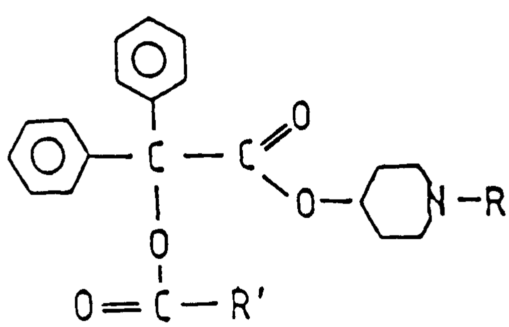

(I)

où R représente H, $CH_3$ $C_2$ $H_5$ , $C_3$ $H_7$ ,
R représente H, $NH_2$ , un phényle qui peut être substitué par un groupe carboxyle, un groupe alcoyle en C1 à C6, un groupe hydroxy(alcoyle en C1 à C6), un groupe méthoxy, éthoxy, méthoxyméthyle ou éthoxyméthyle, un groupe aminoalcoyle en C1 à C6, qui peut être substitué dans le radical alcoyle par un groupe $-NH_2$ , -COOH et/ou $-SCH_3$ , un groupe N-acétylamino(alcoyle en C1 à C6) et un groupe selon la formule

-CH=CH-COOR" ou $-(CH_2)_n$ -COOR" ,

où n est un nombre entier allant de 0 à 8 et R" représente H, $CH_3$ , $C_2H_5$ , $C_3H_7$ ou un cation pharmacologiquement acceptable, ou leurs sels avec des acides pharmacologiquement acceptables.

2. Médicament selon la revendication 1, avec des dérivés d'acide benzylique acylés de formule I définie dans la revendication 1, où R représente $CH_3$ , ou un de ses sels avec un acide pharmacologiquement acceptable.

3. Médicament selon la revendication 1 ou 2, avec des dérivés d'acide benzylique acylés de formule I définie dans la revendication 1, où R' a la signification de $-(CH_2)_n$ -COOR" , n vaut 1, 2 ou 3 et R" est défini comme dans la revendication 1, ou un de ses sels avec un acide pharmacologiquement acceptable.

4. Médicament selon la revendication 1 contenant le 4-(N-méthylpipéridyl)-ester de l'acide α,α-diphényl-α-(3-carboxy-propionyloxy)acétique, ou le 4-(N-méthylpipéridyl)-ester de l'acide α,α-diphényl-α-acétoxy-acétique et/ou le (N-méthylpipéridyl)ester de l'acide α,α-diphényl-α-(4-carboxy-butyryloxy)acétique ou leurs sels avec des acides pharmacologiquement acceptables.

5. Médicament selon l'une des revendications 1 à 4 visant à exercer un effet thérapeutique sur la tonicité de la vessie.

6. Dérivés d'acide benzylique sous forme de 4-(N-alcoylpipéridyl)-esters d'acide α,α-diphényl-α-acyloxy-acétiquede formule

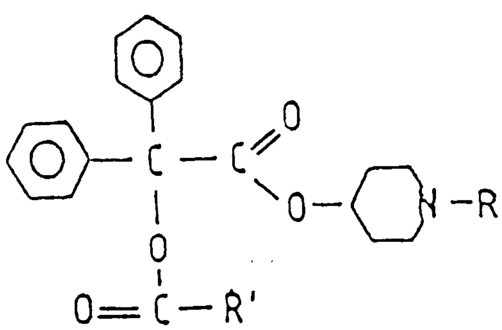

(I)

où R représente H, $CH_3$ , $C_2H_5$ , $C_3H_7$ et R' représente

$-(CH_2)_n$ -COOR" ,

où n est un nombre entier allant de 0 à 8 et R" représente H, $CH_3$ , $C_2H_5$ , $C_3H_7$ ou un cation pharmacologiquement acceptable et leurs sels avec des acides pharmacologiquement acceptables.

7. Dérivés d'acide benzylique selon la revendication 6, où n a une valeur de 0 à 5, en particulier de 1 à 3 et en particulier la valeur de 2, et leurs sels avec des acides pharmacologiquement acceptables.

8. Dérivé d'acide benzylique selon la revendication 6 ou 7, où R représente $CH_3$ , et leurs sels avec des acides pharmacologiquement acceptables.

9. 4-(N-méthylpipéridyl)ester de l'acide α,α-diphényl-α-(3-carboxy-propionyloxy)acétique et 4-(N-méthylpipéridyl)-ester de l'acide α,α-diphényl-α-(4-carboxy-butyryloxy)-acétique et leurs sels avec des acides pharmacologiquement acceptables.

10. Application des dérivés d'acide définis dans les revendications 1 à 5 et 6 à 9 pour exercer un effet thérapeutique sur la tonicité de la vessie.

11. Application des dérivés d'acide benzylique définis dans les revendications 1 à 5 et 6 à 9 à la préparation de médicaments pour exercer un effet thérapeutique sur la tonicité de la vessie.

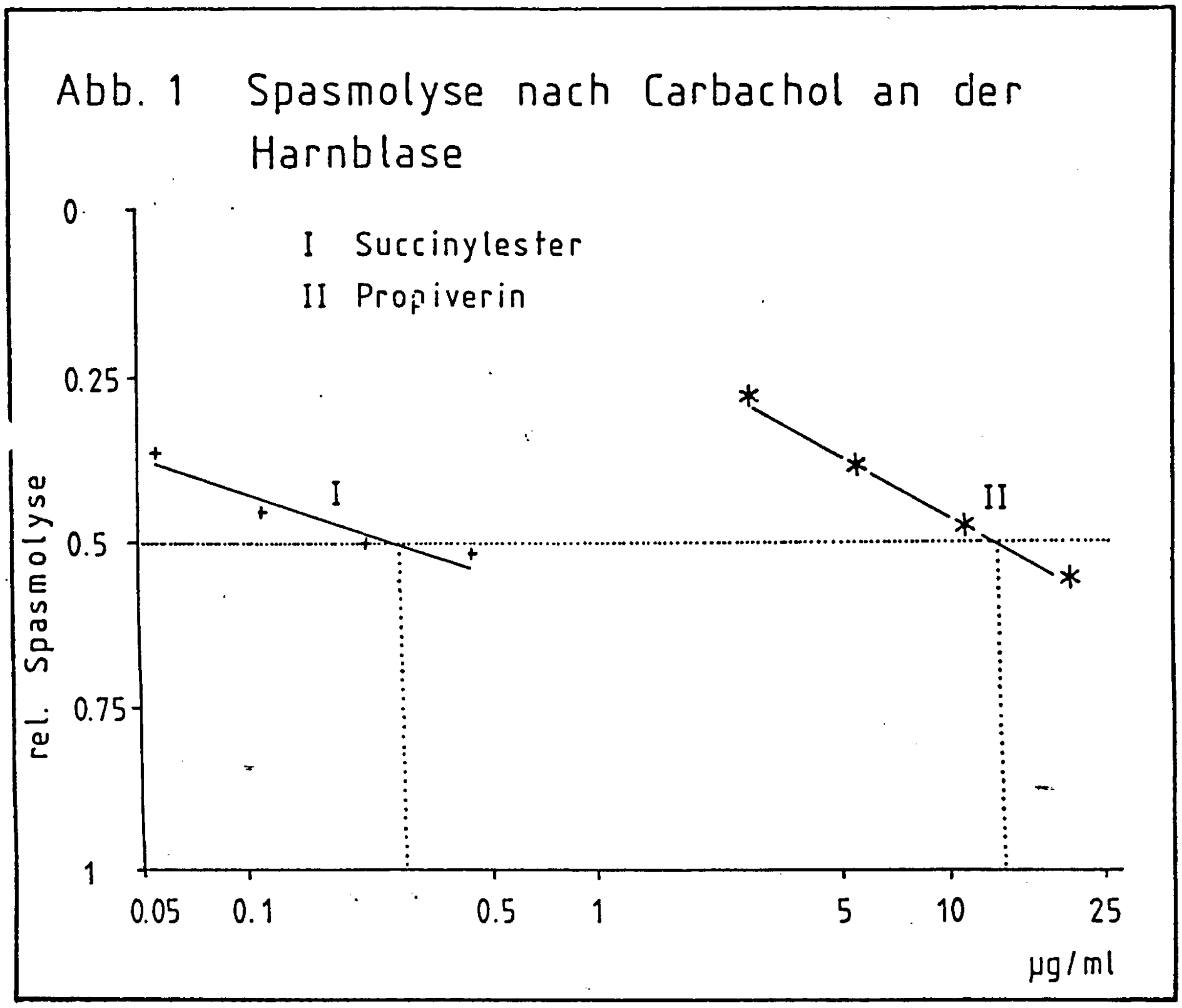
Abb. 1 Spasmolyse nach Carbachol an der Harnblase
I Succinylester
II Propiverin
0
0.25
0.5
0.75
1
rel. Spasmolyse
I
II
0.05
0.1
0.5
1
5
10
25
µg/ml

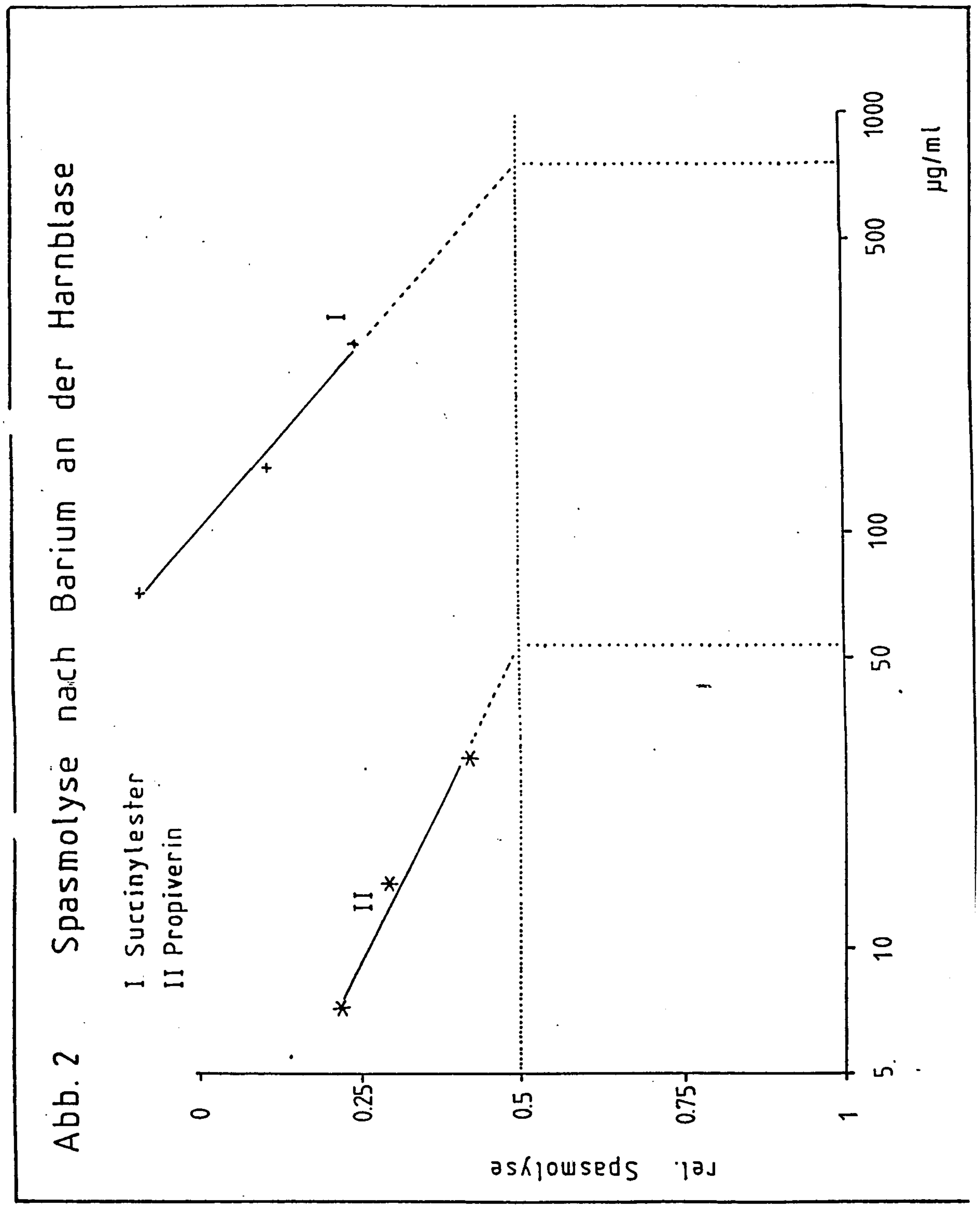
Abb. 2 Spasmolyse nach Barium an der Harnblase
I Succinylester
II Propiverin
I
II
rel. Spasmolyse
0
0.25
0.5
0.75
1
5
10
50
100
500
1000
µg/ml

Abb. 3 Spasmolyse ach Barium an der aenia coli